# FASCICULE DE BREVET EUROPEEN

(11) **EP 4 337 003 B1**
(45) Date de publication et mention de la délivrance du brevet: **22.10.2025**
(21) Numéro de dépôt: 22727373.7
(22) Date de dépôt: 09.05.2022
(51) Int. Cl.: A01K 67/36

(54) **INSTALLATION POUR LA PRODUCTION ET LA COLLECTE DE LARVES NÉONATES**
ANLAGE ZUR PRODUKTION UND SAMMLUNG VON NEONATENLARVEN
FACILITY FOR THE PRODUCTION AND COLLECTION OF NEONATE LARVAE

(30) Priorité: 10.05.2021 FR 2104933
(43) Date de publication de la demande: 20.03.2024
(73) Titulaire: Innovafeed, 80190 Nesle (FR)
(72) Inventeur: OGGERI, Bastien, 75011 PARIS (FR); SCHULLER, Audrey, 94130 NOGENT-SUR-MARNE (FR); TIXIER, Antonin, 92000 NANTERRE (FR); DE VOS, Antoine, 75018 PARIS (FR)
(74) Mandataire: IP Trust
(86) Numéro de dépôt international: PCT/FR2022/050881
(87) Numéro de publication internationale: WO 2022/238646

(56) Documents cités:
- WO-A1-2016/062979
- WO-A1-2019/154563
- FR-A1- 2 460 617
- US-A1- 2020 323 173

## Description

### Domaine de l'invention

La présente invention concerne le domaine de l'élevage industriel d'arthropodes, notamment d'insectes à des fins de production d'aliments.

L'invention se rapporte plus particulièrement au domaine de l'élevage d'insectes, en particulier à la mouche soldat noir.

Les insectes ont un certain nombre de caractéristiques qui les rendent bien adaptés à une utilisation dans l'alimentation animale. Les insectes ont en effet une teneur élevée en protéines, tout en étant riches en autres nutriments bénéfiques tels que les graisses, les minéraux et les vitamines. Les niveaux de concentration de protéines dans les farines d'insectes destinées à l'alimentation animale varient entre 55% et 75%. Les insectes sont caractérisés par un taux de conversion alimentaire plus élevé et peuvent donc devenir une source d'alimentation très précieuse pour les animaux d'élevage. Les insectes sont un composant naturel de l'alimentation des animaux tels que les poissons carnivores et la volaille (par exemple, les insectes peuvent fournir jusqu'à 70% des besoins alimentaires de la truite).

Par ailleurs, ces produits présentent également un profil nutritionnel bien équilibré pour répondre aux besoins alimentaires de l'homme.

Ces considérations ont conduit au développement de la production automatisée en grande série d'aliments issus de l'élevage d'arthropodes, et plus particulièrement d'insectes, dans des sites industriels organisés en espaces complémentaires spécialisés dans l'éclosion, l'élevage, la collecte des animaux matures et leur traitement pour extraire les composés d'intérêt.

Ces sites industriels doivent être optimisés pour permettre l'industrialisation en grands volumes de larves. Une des étapes critiques concerne la collecte des œufs pondus par les insectes femelles et la concentration de larves en vue de leur élevage car il s'agit d'êtres vivants de très petites dimensions, fragiles, très dispersés au départ, et qu'il convient de regrouper de façon aussi homogène que possible, par lots de larves néonates ayant toutes le même stade de maturité dans un lot donné. On entendra par « néonates » au sens du présent brevet les jeunes larves provenant d'œufs fraichement éclos. Généralement, la ponte s'effectue dans des cages confinant les mouches dans un espace fermé dans lesquelles sont disposés des collecteurs présentant des surfaces de ponte, par exemple des plaques rainurées, sur lesquelles les femelles déposent les œufs. Ces collecteurs sont récupérés pour ensuite permettre l'éclosion des œufs donnant lieu à des larves néonates qui sont rassemblées dans des récipients, à des stades aussi homogènes que possible, avant d'être réparties sur un milieu d'élevage dans des modules d'élevage. L'étape qui fait l'objet du présent brevet concerne l'éclosion des œufs et le regroupement des larves néonates.

La présente invention concerne un système permettant l'éclosion d'œufs, préalablement collectés sur des collecteurs, puis le transport et le dosage des néonates obtenus suite à l'éclosion des œufs, avant leur inoculation dans un milieu d'élevage pour permettre leur croissance.

### État de la technique

On connait dans l'état de la technique différentes solutions pour la collecte industrielle et le regroupement dans un récipient de larves néonates, notamment d'insectes.

La demande de brevet internationale WO2019154563A1 décrit un procédé d'élevage et de collecte de jeunes larves, en particulier de la mouche soldat noire, comprenant la mise en place d'insectes dans une cage, comportant des moyens de dépôt d'œufs, le guidage de larves, ayant éclos à partir des œufs déposés, au moyen d'un dispositif de guidage, placé sous la cage, sous l'influence de la gravité vers une courroie transporteuse placée sous les moyens de guidage, le déplacement des larves au moyen de la courroie transporteuse vers un récipient placé à l'extrémité de la courroie transporteuse, le comptage du nombre de larves sur la courroie transporteuse avant la collecte dans le récipient, et la collecte des larves de la courroie transporteuse dans le récipient jusqu'à ce qu'un nombre prédéterminé de larves ait été atteint.

Le document FR2460617 décrit une installation de production en masse d'œufs d'un insecte, notamment du genre pyrale de la farine, du type comportant un incubateur renfermant des éléments à alvéoles, un éclosoir et un pondoir, caractérisée par le fait que l'incubateur est constitué par une enceinte ventilée comportant des supports mobiles d'empilages d'éléments à alvéoles disposés selon leur plan horizontal, un éclosoir destiné à contenir des supports mobiles d'empilage desdits éléments' parallèlement et sur chant au droit d'une trémie de sortie et muni d'une entrée d'anhydride carbonique, et un pondoir comportant un arrangement de feuillets parallèles disposés sur chant au droit d'une trémie de sortie vers un organe récolteur, l'éclosoir, le pondoir et le récolteur étant reliés successivement par un conduit de transport pneumatique, l'incubateur, l'éclosoir et le pondoir étant en outre munis d'organes de régulation de température et, éventuellement, de ventilation.

### Solution apportée par l'invention

La présente invention vise à remédier aux inconvénients des solutions connues dans l'état de la technique en proposant une solution nouvelle adaptée à une production industrielle de masse, à grande échelle, tout en limitant les pertes de production.

A cet effet, l'invention concerne selon son acception la plus générale une installation pour la production et la collecte de larves néonates comportant un ensemble de cages munies d'au moins un collecteur d'œufs pondus par les insectes présents dans lesdites cages, une ligne de transfert des larves néonates issues des œufs éclos depuis chacun desdits collecteurs vers un convoyeur et un équipement de concentration des larves néonates provenant dudit convoyeur pour l'acheminement vers un module d'élevage ou vers un récipient de stockage tampon de larves néonates,
caractérisée en ce qu'elle comporte en outre :
- un ensemble de baies de regroupement d'une pluralité desdits collecteurs d'œufs, lesdites baies étant ouvertes dans leurs parties inférieures ;
- lesdites baies étant mobiles entre un point de chargement avec des collecteurs retirés des cages et ladite ligne de transfert des larves néonates ;
- ladite ligne de transfert des larves néonates étant ouverte, sans carter dans la partie supérieure, pour recevoir lesdites baies en partie supérieure, et présentant en partie inférieure un système de guidage à parois évasées, dit réceptacle, présentant dans sa partie supérieure une largeur adaptée à la largeur desdites baies, et dans sa partie inférieure une ouverture adaptée aux dimensions dudit convoyeur, ledit réceptacle étant configuré pour regrouper les larves néonates tombant par gravité desdits collecteurs sur la surface supérieure dudit convoyeur.

Avantageusement, ledit convoyeur comprend au moins une bande défilante, positionnée sous l'ouverture inférieure dudit réceptacle à parois évasées.

Selon une variante, ledit convoyeur comprend un couloir vibrant positionné sous l'ouverture inférieure dudit réceptacle à parois évasées.

Selon une variante, ledit réceptacle à parois évasées est constitué par une gouttière fendue présentant deux flans latéraux dont les bords inférieurs sont séparés par une fente longitudinale.

Avantageusement, ledit réceptacle à parois évasées comporte en partie supérieure des moyens de déplacement longitudinal desdites baies.

Selon un mode de réalisation particulier, ladite ligne de transfert comporte en partie supérieure du réceptacle à parois évasées des moyens de déplacement desdites baies parallèles à ladite bande défilante.

Avantageusement, ladite bande défilante présente une section principale horizontale prolongée à son extrémité aval par une section inclinée en direction d'un système d'aspiration, un moyen vibratoire agissant sur la section supérieure et/ou sur la section inférieure.

De préférence, ladite bande défilante présente une section principale horizontale prolongée à son extrémité aval par une section inclinée en direction d'un système d'aspiration, la bande inférieure de cette section étant soumise à un flux d'air dirigée dans le sens opposé au défilement de ladite bande inférieure.

Selon un mode de mise en œuvre particulier, l'installation comporte un système de transport pneumatique par aspiration pour l'acheminement des larves néonates depuis ledit convoyeur vers un moyen de distribution desdites larves néonates.

Selon une variante préférée, l'installation comporte un séparateur cyclonique formé par un cylindre et/ou un cône vertical dans lesquels un flux d'air ascendant est produit, et dont le fond comporte un moyen de décharge mécanique et périodique.

Avantageusement, ledit moyen de décharge mécanique et périodique est constitué par une écluse à pales rotatives.

De préférence, l'installation comporte également un moyen de dosage de la quantité de néonates par pesée.

De préférence, l'installation comporte un système d'aiguillage pour la distribution des larves néonates collectées sur les plateaux d'un module multi-étagé.

L'invention concerne aussi un procédé d'élevage et de collecte des larves néonates d'arthropodes comprenant les étapes suivantes :
a) disposer d'un ensemble de cages remplies d'insectes, munies d'au moins un collecteur d'œufs permettant aux insectes femelles de déposer leurs œufs ;
b) recueillir les néonates sous l'influence de la gravité vers un convoyeur assurant le déplacement des larves néonates au moyen du convoyeur vers un moyen de recueil ;
caractérisé en ce que
l'on procède à une étape de regroupements d'une pluralité de collecteurs dans des baies et à une étape de déplacement desdites baies sur une ligne de transfert ouverte comportant une gouttière fendue présentant deux parois évasées convergeant définissant une fente de déversement des larves néonates sur ledit convoyeur vers l'extrémité aval de récolte desdites larves néonates.

Avantageusement, l'introduction d'une nouvelle baie sur la ligne de transfert fait progresser les baies déjà positionnées dans ladite installation en direction avale.

L'invention concerne également une baie pour l'accrochage de collecteurs d'œufs caractérisée en ce qu'elle est constituée par une armature ouverte formée d'un assemblage rigide, sur laquelle sont prévus des rails à galets transversaux entre lesquels peuvent être insérés des cadres supportant chacun une pluralité de collecteurs d'œufs issus des cages de ponte.

### Description détaillée d'exemples non limitatif de réalisation

La présente invention sera mieux comprise à la lecture de la description qui suit, se référant aux dessins annexés illustrant des exemples non limitatifs de réalisation où :
[FIG. 1] la figure 1 représente une vue de dessus schématique d'ensemble d'une installation pour la production et la collecte de larves néonates selon l'invention ;
[FIG. 2] la figure 2 représente une vue en perspective schématique d'ensemble d'une installation pour la production et la collecte de larves néonates selon l'invention ;
[FIG. 3] la figure 3 représente une vue en perspective d'une baie pour la réception des collecteurs de ponte ;
[FIG. 4] la figure 4 représente une vue de côté de deux baies et d'une ligne de transfert ;
[FIG. 5] la figure 5 représente une vue en perspective de l'extrémité aval de la bande défilante ;

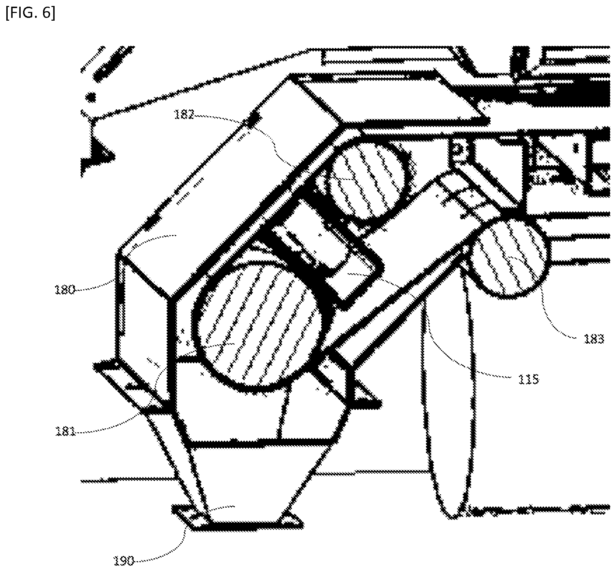

[FIG. 6] la figure 6 représente une vue en perspective agrandie de l'extrémité aval de la bande défilante ;
[FIG. 7] la figure 7 représente une vue en perspective de la trémie de distribution des larves ;
[FIG. 8] la figure 8 représente une vue en perspective du système d'aiguillage.

Dans la description qui suit, certains détails sont développés en référence à un équipements, et dans un passage. Cela n'exclut pas que les mêmes détails sont aussi présents dans l'équipement mentionné dans un autre passage, même dans un passage concernant une autre variante de réalisation, du simple fait que les détails en question n'ont pas fait l'objet d'une nouvelle description.

Dans le présent brevet, le terme «comprenant», utilisé dans les revendications, ne doit pas être interprété comme étant limité aux éléments ou étapes énumérés ci-après; il n'exclut pas d'autres éléments ou étapes. Il doit être interprété comme spécifiant la présence des caractéristiques, des étapes ou des composants mentionnés, mais n'exclut pas la présence ou l'ajout d'une ou plusieurs autres caractéristiques, étapes ou composants, ou groupes de ceux-ci. Ainsi, la portée de l'expression «un dispositif comprenant A et B» ne doit pas être limitée aux dispositifs constitués uniquement des composants A et B, mais en ce qui concerne la présente invention, les seuls composants énumérés du dispositif sont A et B, et en outre, l'allégation doit être interprétée comme incluant des équivalents de ces composants.

### Principes généraux

La production industrielle d'aliments provenant de l'élevage d'arthropodes et - dans l'exemple décrit à titre non limitatif - d'insectes se fait dans des installations en grande partie automatisées destinées à créer les conditions optimales pour permettre massivement le passage du stade d'évolution de l'œuf, de l'oothèque ou de larve néonate à l'insecte adulte, en passant par la larve (ou asticots ou aptères) et la nymphe ou pupe.

Typiquement, ces installations sont organisées en plusieurs bâtiments comportant des rayonnages pour le stockage des modules d'élevage chargés d'un milieu d'élevage inoculé de larves dans une atmosphère climatique propice à l'élevage, et des équipements de manipulation pour les traitements épisodiques.

L'invention vise à améliorer le rendement de la zone allant de l'éclosion des œufs jusqu'à l'inoculation des néonates dans leur milieu d'élevage. Pour ce faire, le premier enjeu est d'avoir le meilleur taux d'éclosion possible, c'est-à-dire que la plupart des œufs éclosent et deviennent des néonates.

Ensuite, le second enjeu est d'assurer un transport efficace des néonates jusqu'à leur contenant de croissance, sans qu'ils puissent s'échapper du système.

Enfin, le dernier enjeu est d'assurer une quantification précise de néonates du même âge afin de limiter les variabilités dans le processus. En effet, cette étape est primordiale pour la suite du cycle d'élevage. S'il y a trop de néonates par rapport à la quantité de milieu d'élevage, alors leur croissance ne sera pas optimale car ils n'auront pas assez de nourriture. A l'inverse, s'il n'y a pas assez de néonates, le milieu d'élevage ne sera pas intégralement consommé et cela posera des problèmes pour les étapes aval du cycle, notamment pour l'étape de séparation des larves et de leur milieu d'élevage. De la même manière, si les néonates ont des âges différents, il sera difficile de bien contrôler leur croissance.

Un autre enjeu transversal est d'avoir des technologies fiables, robustes, peu coûteuses, et le moins gourmandes possible en consommations, par exemple en air comprimé.

### Processus de collecte

Le processus de collecte s'inscrit entre le processus de ponte, qui s'effectue en amont de la collecte dans des cages de ponte, et le processus d'élevage qui s'effectue en aval de la collecte, dans des modules remplis de milieu d'élevage.

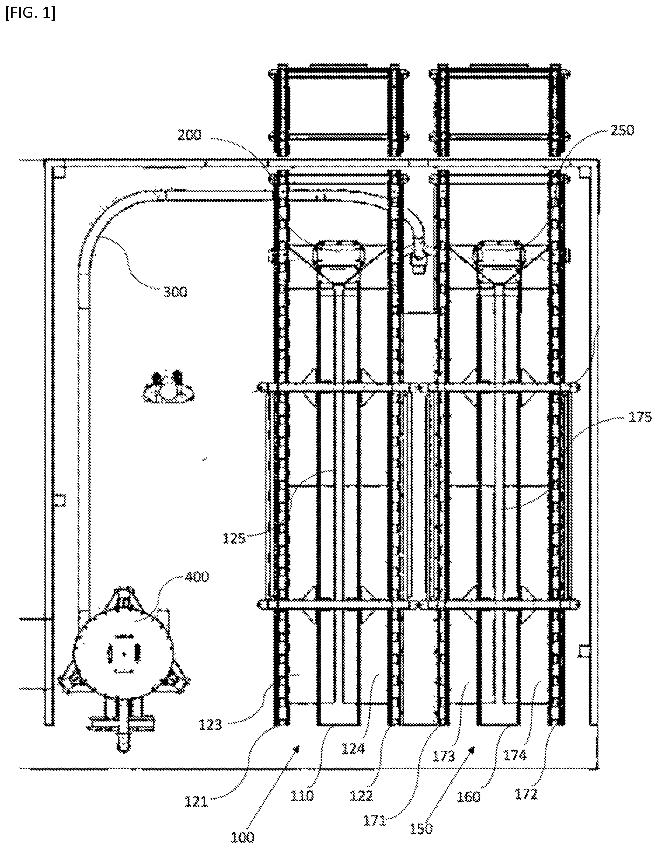

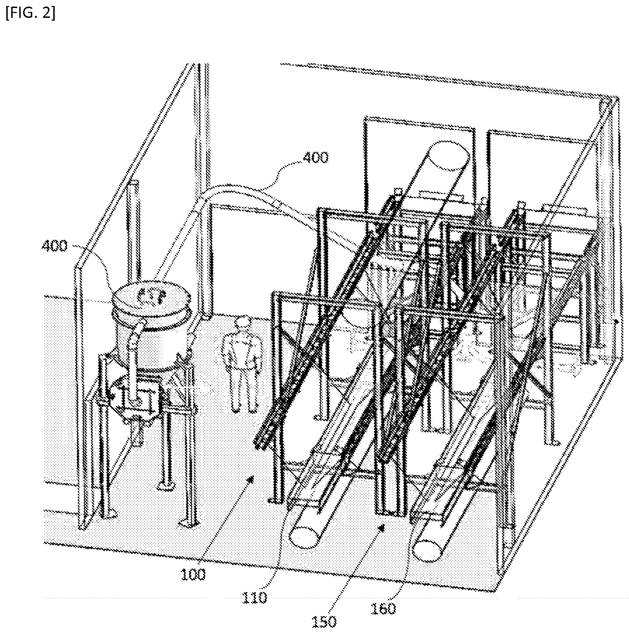

Les étapes du processus de collecte se font dans une seule et même zone, par exemple un bâtiment ou un hangar, dans laquelle se trouvent des équipements illustrés par les figures 1 et 2. Dans cette zone règne une ambiance climatique contrôlée propice à l'éclosion de œufs, sauf la partie inoculation qui se fait dans une zone différente.

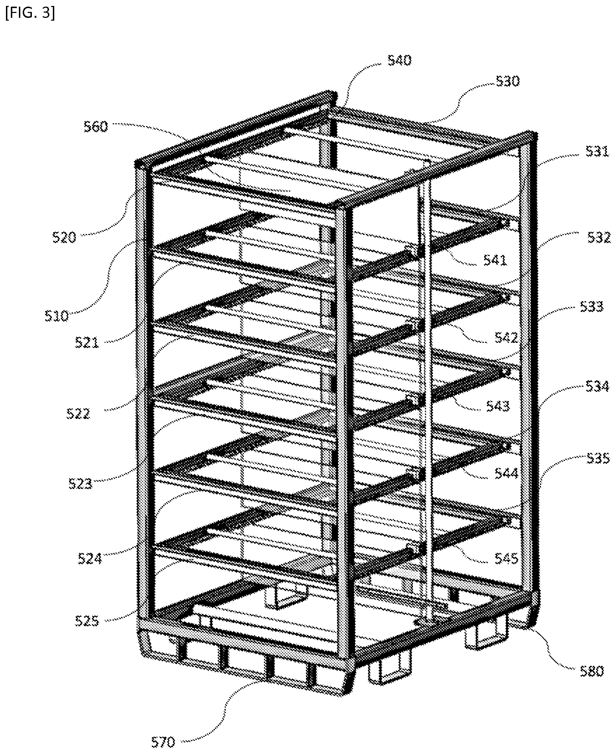

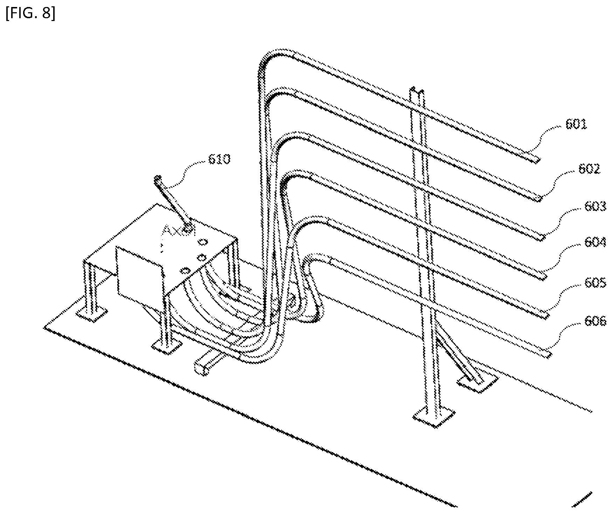

Les équipements mise en œuvre pour ces étapes comprennent principalement :
- une pluralité de baies illustrées par la figure 3, destinées à recevoir les collecteurs extraits des cages ;
- des lignes de transfert des larves néonates (100, 150) comportant des rails de roulement à galets (121, 122 ; 171, 172) destinés au déplacement longitudinal des baies (500, 501) susvisées, et des bandes défilantes (110, 160) ;
- un système d'aspiration (300) pour le transfert vers un séparateur cyclonique (400) ;
- un système de distribution pour alimenter les modules d'élevage avec des larves néonates, illustré par la figure 8

Les œufs arrivent dans la zone d'éclosion sur des collecteurs provenant d'une autre zone dans laquelle les mouches ont préalablement pondu sur les collecteurs, généralement dans une cage. Ces collecteurs se présentent par exemple sous forme de plaques rainurées. Ils sont disposés sur des baies présentant des rails à galets de réception de collecteurs conçues spécifiquement pour permettre d'avoir une forte densité d'œufs (et donc ensuite de néonates), sans créer d'interférence lors de la chute gravitaire des néonates, le tout en étant facilement manipulables par des engins de manutention standards.

Le système de support et de transport de ces baies de collecteurs sur les lignes de transfert (100, 150) laisse le temps adéquat pour que tous les œufs éclosent. Pour ce faire, les baies de collecteurs sont déplacées à l'aide d'un transport mécanique passif selon une logique FIFO (la dernière baie de collecteurs arrivée chasse la plus ancienne de la zone).

Une fois éclos, les néonates tombent par gravité sur des bandes défilantes (110, 160). Au cours de leurs chutes, les néonates tombent dans une gouttière fendue et sont guidés par des flans latéraux (123, 124 ; 173, 174) formant des plans inclinés débouchant sur des fentes longitudinales (125, 175) située à l'aplomb des bandes défilantes (110, 160). Les néonates étant collant, il est important de bien choisir le matériau de la surface des gouttières pour limiter autant que possible l'adhérence des néonates à cette surface. Typiquement, ces gouttières fendues sont réalisées par formage de tôles d'acier Inoxydable poli miroir, c'est-à-dire avec une rugosité inférieure à 0,2 microns.

Les néonates sont ensuite récupérés à la fin des bandes défilantes (110, 160), mais sans être stockés à cet endroit. Les néonates étant « collants », une configuration spécifique pour bien les décoller en fin des bandes défilantes (200, 250) est prévue et est décrite plus en détail ci-après.

Pour augmenter encore la production les lignes de transfert et équipements associés peuvent être répliqués autant de fois que nécessaire et fonctionner en parallèle.

A la fin des bandes défilantes, la chaine d'automatisation n'est pas rompue. Les néonates sont alors transportés pneumatiquement jusqu'à un séparateur cyclonique (400). Celui-ci peut travailler indifféremment en surpression ou en dépression. Un système mécanique simple permet d'ouvrir et de fermer périodiquement ce séparateur cyclonique (400) afin d'acheminer les néonates à la prochaine étape.

Après le séparateur cyclonique (400) et après son système d'ouverture et de fermeture périodique, par exemple une écluse à pales rotatives, une zone tampon est nécessaire. Elle permet ainsi de pouvoir stocker la quantité de néonates nécessaire afin de produire en continu et de pouvoir assurer une alimentation continue en néonates des phases aval du processus.

Les différents éléments de l'installation sont décrits ci-après à titre d'exemple, de manière plus détaillée. Ces différents éléments peuvent être combinés entre eux, ou combinés avec d'autres éléments remplissant la même fonction pour former une installation selon l'invention.

### Détail du collecteur d'œuf

Les collecteurs ont une forme rectangulaire de dimensions 850 mm x 230 mm. Leurs deux faces sont rainurées. C'est dans ces rainures que les mouches femelles viennent pondre durant l'étape préalable de ponte. Après que les mouches aient pondu, les collecteurs sont récupérés, par exemple par un opérateur manutentionnaire, et sont regroupés sur des baies (500, 501) de collecteurs.

### Détail de la baie

Une baie est constituée par une armature parallélépipédique (510) ouverte formée d'un assemblage de tubes soudés rigides, sur laquelle sont prévues des glissières, par exemple formées par des rails à galets transversaux (520 à 525 ; 530 à 535) entre lesquels peuvent être insérées des cadres (540 à 545) supportant chacun une pluralité de collecteurs d'œufs (560) issus de la cage de ponte.

Ces baies (500, 501) de collecteurs peuvent supporter jusqu'à 240 collecteurs. La forme globale des baies de collecteurs est parallélépipédique de dimensions 1200 mm x 1000 mm x 1800 mm, la structure est assurée par un châssis extérieur laissant ainsi toute la place nécessaire au centre pour que les néonates puissent tomber par gravité une fois les œufs éclos. Les collecteurs sont suspendus à des profilés traversant le châssis de la baie de collecteurs grâce à des vis. L'armature reposent sur des patins (570, 580).

Les bases des baies ont une forme bien particulière afin de permettre aisément leur manipulation par de classiques transporteurs mais aussi pour pouvoir être compatibles avec l'utilisation d'un transporteur à galets.

Les baies (500, 501) de collecteurs (560) sont stockées dans la zone à l'ambiance climatique contrôlée sur des rails de roulements à galets (121, 122 ; 171, 172).

Une baie est chargée avec des cadres (540 à 545) sur lesquels ont été accrochée une série de collecteurs d'œufs (560) fraichement retirés de la cage de ponte, simultanément (au temps de manipulation près) pour que tous les collecteurs contiennent des œufs de même maturité, l'écart de maturité étant dépendant du rythme de collecte des collecteurs. Ce temps ne dépasse généralement pas quelques heures.

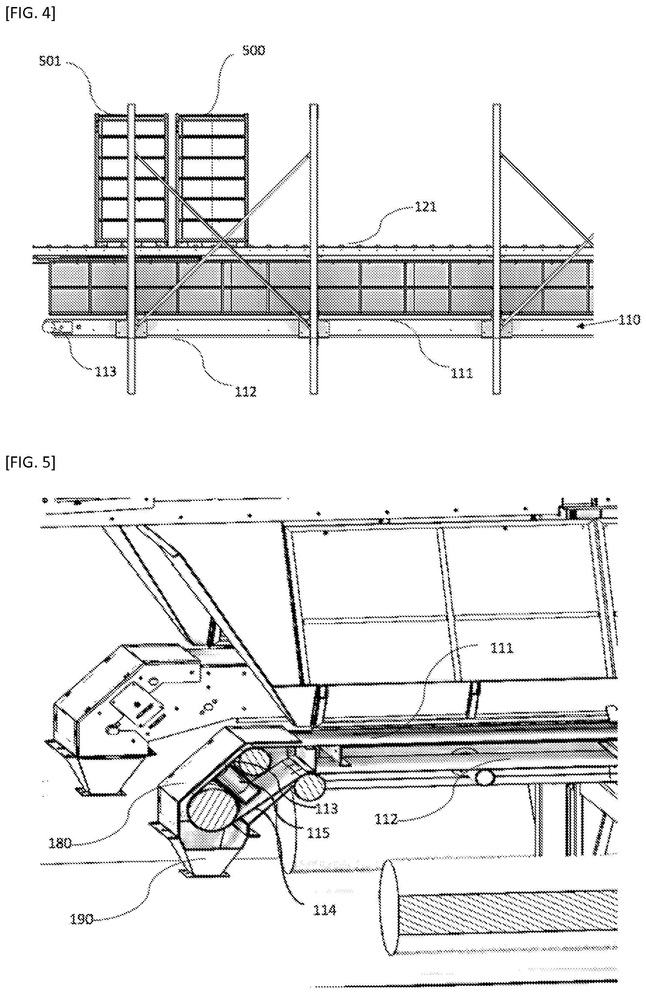

Une fois chargée, elle est déplacée jusqu'à la ligne de transfert (100, 150) et déposée sur les rails de roulement à galets (121, 122 ; 171, 172). Dans l'exemple illustré par la figure 4, l'introduction d'une nouvelle baie repousse vers l'aval la ou les baies (500) déjà placées sur la ligne de transfert. Il est possible de déplacer les baies (500, 501) manuellement, en repoussant avec la dernière baie (501) la série de baies déjà posés sur les rails de roulement à galets (121, 122 ; 171, 172), ce qui fait progresser l'ensemble, et la baie la plus ancienne est récupérée à l'extrémité aval des lignes de transfert (100, 150) pour être ensuite retournée vers la zone où se trouvent les cages de ponte pour être rechargée avec de nouveaux collecteurs d'œufs

(560).

La bande défilante (110) forme une boucle avec un segment supérieur (111) disposé sous la fente s'ouvrant entre les deux flans inclinés, et un segment inférieur (112) pour le retour de la bande qui défile grâce à un rouleau amont (113).

Pendant le séjour d'une baie sur la ligne de transfert, les larves néonates tombent naturellement lorsque les œufs éclosent et sont regroupées, grâce à la gouttière fendue, sur la surface de la bande défilante (110, 160) qui avance à une vitesse permettant de concentrer les larves posées à sa surface pour les amener à un système d'aspiration prévu à l'extrémité avale du convoyeur.

### Détail de la partie amont de la ligne de transfert

La ligne de transfert est constituée par un bâti supportant des rails à galets (121, 122 ; 171, 172) pour le déplacement des baies (500, 501) au-dessus d'une gouttière fendue ramenant les larves néonates qui tombent des collecteurs (560) sur la surface d'une bande défilante. Le bâti comporte un mécanisme motorisé assurant l'entraînement de cette bande par l'intermédiaire de rouleaux d'entrainement.

Les néonates ne doivent pas exagérément collés à la bande défilante au risque d'être trop difficiles à décoller. A l'inverse, il faut qu'ils adhèrent un minimum pour ne pas tomber de la bande sur les côtés et être bien transportés. Un matériau bien approprié est le polyuréthane non adhésif.

La longueur utile des bandes défilantes (110, 160) est de 15 m pour correspondre à la longueur du convoyeur à galets situé au-dessus, elle-même déterminée par le temps de séjour et le nombres de baies pour assurer la production visée.

La largeur des bandes défilantes est de 400 mm : une bande trop étroite favoriserait les échappés de néonates par les côtés, une bande trop large rendrait leurs décollements en fin de bande plus difficile à gérer.

La bande se déplace à une vitesse nominale de 0,1 m/s dans le même sens que le sens de déplacement des baies. Les œufs éclosant majoritairement à la fin de le leur séjour dans la zone, les néonates tomberont ainsi majoritairement à la fin des bandes défilantes.

Le stockage de ces baies (500, 501) est réalisé en FIFO (First In First Out). La dernière baie (500, 501) de collecteurs qui arrive sur le convoyeur à galets pousse vers l'extérieur de la zone celle qui était rentrée dans la zone depuis le plus longtemps. Le déplacement des baies est donc un déplacement mécanique passif.

Les baies restent en moyenne 4 jours dans la zone. Ce temps est déterminé pour que l'ensemble des œufs aient le temps d'éclore et que les néonates tombent par gravité sur la bande défilante située sous le convoyeur à galets.

Le convoyeur à galets comporte 15 emplacements de baies.

Pour guider les néonates dans leurs chutes, des plans inclinés à 55°ont été installés.

### Détail de la partie aval de la ligne de transfert

La bande défilante (110, 160) de la ligne de transfert se termine par un segment avec une partie supérieure (113) et une partie retour (114) en pente, avec une inclinaison de 45° sur une longueur de 260 mm protégée des courants d'air par une tôle formant un auvent (180).
Cette pente a un double avantage :
- elle augmente le temps de séjour des néonates dans la zone où ils doivent être décollés, cela augmente donc considérablement le taux de décollement ;
- les néonates en amont qui sont déjà décollés glissent sur la bande et entraînent avec eux des néonates situés en aval et éventuellement ceux qui n'étaient pas encore décollés.

Cette modification de l'orientation de la bande défilante est réalisée par un système de rouleaux (181, 182, 183).

Un premier percuteur (115) est installé à la fin de la bande défilante, dans sa partie inclinée, afin de faire vibrer le segment supérieur (113) de la bande et d'aider au décollement des néonates. Il s'agit par exemple d'un vibreur électromagnétique vibrant à une fréquence de quelques dizaines de Hertz et avec une amplitude de quelques millimètres.

Pour une meilleure efficacité, un deuxième percuteur est ajouté juste après, dans la zone entre le premier percuteur et le rouleau de retournement (181). Dans cette zone, la tension de la bande est la plus élevée, ce qui permet des vibrations plus efficaces pour favoriser le décollement.

Un système aéraulique produit un jet d'air à contrecourant, par rapport à la direction de défilement de la partie de retour (114) du segment incliné afin de décoller les néonates qui pourraient rester collés. Plus le soufflage est fort, plus la fonction de décollement sera efficace. En revanche, le soufflage doit rester limité pour ne pas créer un flux d'air trop important sur la face aller de la bande défilante et risquer de créer des envols et donc des pertes de néonates sur la face aller de la bande défilante. Un système de racleur ou de brosse peut également être ajouté afin de décoller les néonates.

A l'extrémité aval de la bande défilante, l'ensemble est capoté au plus proche. Les néonates étant très légers, le moindre flux d'air parasite peut venir perturber le système, il est donc important de bien capoter l'ensemble, pour favoriser des flux d'air préférentiels et pour éviter des flux d'air parasites.

Enfin, en fin de bandes défilantes, une bouche de jonction (190) assure le capotage du système pour permettre de transporter les néonates par transport pneumatique jusqu'à un séparateur cyclonique (400), à une vitesse d'air comprise entre 5 et 10 m/s. Cette pièce de jonction a une forme d'entonnoir afin d'éviter les rétentions.

Dans cet exemple de l'invention, cet ensemble constitué du convoyeur à galets, de la gouttière, des plans inclinés et de la bande défilante est installé deux fois en parallèle pour avoir une production doublée. Les néonates provenant de ces deux ensembles en parallèle sont en revanche transportés pneumatiquement jusqu'au même séparateur cyclonique (400).

### Système d'aspiration

Le séparateur cyclonique (400) est formé par un cylindre vertical à double paroi, de diamètre 950 mm. Ce séparateur cyclonique (400) permet une vitesse ascensionnelle limitée de 0,25 m/s. Cette vitesse doit être faible pour éviter que les néonates ne remontent.
Ce séparateur cyclonique se termine par une écluse à pales rotatives. Celle-ci tourne en continu, alternant les phases où les néonates se déversent et les phases où les néonates sont bloqués dans le séparateur cyclonique. Le design spécifique de l'écluse fait que cette séparation mécanique n'endommage pas ou n'écrase pas les néonates. Cette écluse est en inox pour éviter l'adhérence. Pour éviter l'écrasement des néonates, cette écluse possède 6 pâles qui est le nombre minimal permettant d'assurer une bonne étanchéité. Elle tourne à une vitesse réduite de 6 tr/min.

### Doseur et couloir vibrant sur pesons

Après ce séparateur cyclonique, une étape de dosage est nécessaire afin d'avoir la quantité de néonates souhaitée pour que l'aval du processus soit réalisé de manière optimale. Ici, le choix a été fait de quantifier la quantité de néonates par pesée, contrairement à l'art antérieur qui utilise un comptage optique. La pesée a le double avantage d'être facile à mettre en œuvre et d'être suffisamment précise par rapport au besoin. De plus, compte tenu des quantités de néonates visées pour l'invention, le comptage optique peut s'avérer limitant, soit à cause du temps de traitement des images, soit si le flot de néonates est ininterrompu sur la bande défilante.

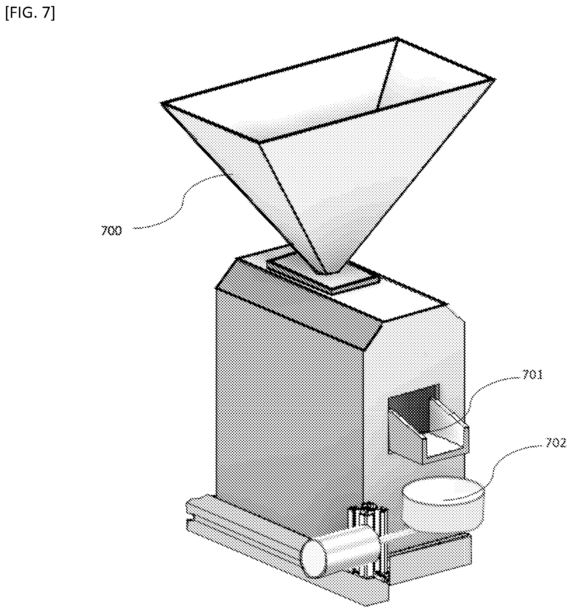

L'unité de pesage représentée en figure 7 comporte une trémie (700) en partie supérieure en forme d'entonnoir pour recueillir les néonates issues du séparateur cyclonique (400). Ces néonates tombent sur le couloir vibrant (701). Par vibration, ce couloir (701) déverse les néonates petit à petit dans le godet retourneur (702) jusqu'à atteindre la masse souhaitée de larves néonates dans ce godet.

Le volume de cette trémie (700) en forme d'entonnoir, servant de zone tampon, est défini pour garantir une bonne fluidité de la production. Le couloir vibrant (701) est actionné par un système de vibration afin d'éviter le colmatage et d'aider les néonates à s'écouler.

Dès qu'un des plateaux d'un module multi-étagé est prêt à être inoculé, le godet (702) est basculé dans un entonnoir et les néonates sont transportés pneumatiquement vers ce plateau via un système d'aiguillage.

L'intérêt de ce godet retourneur (702) est de pouvoir directement commencer la pesée de la dose suivante pendant que l'inoculation est en cours. Cela permet ainsi de paralléliser les opérations et donc d'avoir un meilleur cadencement de la production.

### Système d'aiguillage

Ce système d'aiguillage illustré par la figure 8 permet d'inoculer les larves dans l'ensemble des six plateaux d'un module multi-étagé, sans que ce module ne nécessite un déplacement. Cette inoculation se fait de manière séquentielle, chaque plateau étant inoculé l'un à la suite de l'autre.

Le dispositif de distribution comprend 6 rampes (601 à 606) régulièrement espacés avec un pas correspondant à l'intervalle des plateaux d'un module d'élevage multi-étagé. La longueur de ces rampes est déterminée en fonction des dimensions des plateaux afin de permettre la répartition des larves néonates sur la surface du milieu d'élevage garnissant le plateau.

Le fonctionnement de ce système d'aiguillage à 6 voies est basé sur le mouvement de la goulotte mobile (610) située directement en aval de l'entonnoir. Cette goulotte mobile (610) change de position pour se connecter à une des rampes (601 à 606) constituant les goulottes nourrices de chaque plateau l'une après l'autre. Une dose peut être faite toutes les 26 secondes, seulement 156 secondes sont donc nécessaires pour inoculer un module multi-étagé en entier.

### Fonctionnement par « intermittence »

L'ensemble des éléments décrits ci-dessus fonctionne selon une séquence intermittente. En effet, les néonates tombant au fur et à mesure sur les bandes défilantes, il n'est pas nécessaire de le faire tourner en continu. Cependant, il n'est pas non plus possible de l'arrêter de manière prolongée pour éviter que les néonates s'échappent par les côtés. La séquence intermittente optimale a été définie de manière empirique par une période de fonctionnement de 8 minutes puis un arrêt de 5 minutes et ainsi de suite.

Le principal intérêt de ce fonctionnement par intermittence est de réaliser des économies d'énergies et de réduire les consommations (notamment l'air comprimé) sans avoir le moindre impact sur la production, notamment grâce au stockage tampon.

## Revendications

1. Installation d'élevage d'arthropodes pour la production et la collecte de larves néonates comportant un ensemble de cages munies d'au moins un collecteur d'œufs (560) pondus par les insectes présents dans lesdites cages, une ligne de transfert des larves néonates issues des œufs éclos depuis chacun desdits collecteurs (560) vers un convoyeur et un équipement de concentration des larves néonates provenant dudit convoyeur pour l'acheminement vers un module d'élevage ou vers un récipient de stockage tampon de larves néonates, **caractérisée en ce qu'**elle comporte en outre :
• un ensemble de baies (500, 501) de regroupement d'une pluralité desdits collecteurs d'œufs (560), lesdites baies (500, 501) étant ouvertes dans leurs parties inférieures ;
• lesdites baies (500, 501) étant mobiles entre un point de chargement avec des collecteurs retirés des cages, et ladite ligne de transfert des larves néonates ;
• ladite ligne de transfert des larves néonates étant ouverte, sans carter dans la partie supérieure, pour recevoir lesdites baies (500, 501) en partie supérieure, et présentant en partie inférieure un système de guidage à parois évasées (123, 124 ; 173, 174), dit réceptacle, présentant dans sa partie supérieure une largeur adaptée à la section desdites baies, et dans sa partie inférieure une ouverture (125, 175) adaptée aux dimensions dudit convoyeur (110, 160), ledit réceptacle étant configuré pour regrouper les larves néonates tombant par gravité desdits collecteurs (560) sur la surface supérieure dudit convoyeur (110, 160).

2. Installation pour la production et la collecte de larves néonates selon la revendication 1 **caractérisée en ce que** ledit convoyeur comprend au moins une bande défilante (110, 160), positionnée sous l'ouverture inférieure dudit réceptacle à parois évasées (123, 124 ; 173, 174).

3. Installation pour la production et la collecte de larves néonates selon la revendication 1 **caractérisée en ce que** ledit convoyeur comprend au moins un couloir vibrant positionné sous l'ouverture inférieure dudit réceptacle à parois évasées (123, 124 ; 173, 174).

4. Installation pour la production et la collecte de larves néonates selon la revendication 1 ou 2 **caractérisée en ce que** ledit réceptacle à parois évasées (123, 124 ; 173, 174) est constitué par une gouttière fendue présentant deux flans latéraux (123, 124 ; 173, 174) dont les bords inférieurs sont séparés par une fente longitudinale (125, 175).

5. Installation pour la production et la collecte de larves néonates selon la revendication 4 **caractérisée en ce que** ledit réceptacle à parois évasées (123, 124 ; 173, 174) comporte en partie supérieure des moyens de déplacement longitudinal desdites baies (500, 501).

6. Installation pour la production et la collecte de larves néonates selon la revendication 2 **caractérisée en ce que** ladite installation comporte en outre une système d'aspiration et **en ce que** ladite bande défilante (110, 160) présente une section principale horizontale prolongée à son extrémité aval par une section inclinée (113, 114) en direction dudit système d'aspiration, un moyen vibratoire (115) agissant sur la section supérieure (113) et/ou sur la section inférieure (113) .

7. Installation pour la production et la collecte de larves néonates selon la revendication 2 **caractérisée en ce que** ladite bande défilante présente une section principale horizontale prolongée à son extrémité aval par une section inclinée en direction dudit système d'aspiration, la bande inférieure (114) de cette section étant soumise à un flux d'air dirigée dans le sens opposé au défilement de ladite bande inférieure (114).

8. Installation pour la production et la collecte de larves néonates selon la revendication 1 **caractérisée en ce qu'**elle comporte un système de transport pneumatique (300) pour l'acheminement des larves néonates depuis ledit convoyeur vers un moyen de distribution desdites larves néonates.

9. Installation pour la production et la collecte de larves néonates selon la revendication 1 **caractérisée en ce qu'**elle comporte un séparateur cyclonique (400) formé par un cylindre et/ou un cône vertical dans lequel un flux d'air ascendant est produit et dont le fond comporte un moyen de décharge mécanique et périodique.

10. Installation pour la production et la collecte de larves néonates selon la revendication précédente **caractérisée en ce que** ledit moyen de décharge mécanique et périodique est constitué par une écluse à pales rotatives

11. Installation pour la production et la collecte de larves néonates selon la revendication 1 **caractérisée en ce qu'**elle comporte un moyen de dosage de la quantité de néonates par pesée.

12. Installation pour la production et la collecte de larves néonates selon la revendication 1 **caractérisée en ce qu'**elle comporte système d'aiguillage pour la distribution des larves néonates collectées sur les plateaux d'un module multi-étagé.

13. Procédé d'élevage et de collecte des larves néonates d'arthropodes comprenant les étapes suivantes :
a) disposer d'un ensemble de cages remplies d'insectes, munies d'au moins un collecteur d'œufs permettant aux insectes femelles de déposer leurs œufs ;
b) recueillir les néonates sous l'influence de la gravité vers un convoyeur assurant le déplacement des larves néonates au moyen dudit convoyeur vers un moyen de recueil ;
**caractérisé en ce que**
l'on procède à une étape de regroupements d'une pluralité de collecteurs dans des baies (500, 501) et à une étape de déplacement desdites baies (500, 501) sur une ligne de transfert ouverte comportant une gouttière fendue présentant deux parois inclinées (123, 124 ; 173 ; 174) convergeant définissant une fente (125, 175) de déversement des larves néonates vers ledit convoyeur vers l'extrémité aval de récolte desdites larves néonates.

14. Procédé d'élevage et de collecte des larves néonates d'arthropodes selon la revendication 13 **caractérisé en ce que** l'introduction d'une nouvelle baie (501) sur ladite ligne de transfert fait progresser les baies déjà positionnées sur ladite ligne de transfert en direction avale.

## Patentansprüche

1. Arthropoden-Zuchtanlage zur Erzeugung und Sammlung von Neonatenlarven, die eine Reihe von Käfigen umfasst, die mit mindestens einem Eiersammler (560) versehen sind, die von den in den Käfigen vorhandenen Insekten gelegt werden, eine Transferlinie der aus den geschlüpften Eiern stammenden Neonatenlarven von jedem der Sammler (560) zu einem Förderer und eine Einrichtung zum Konzentrieren der von dem Förderer kommenden Neonatenlarven für den Transport zu einem Aufzuchtmodul oder zu einem Behälter zur Pufferlagerung der Neonatenlarven, **dadurch gekennzeichnet, dass** sie außerdem umfasst:
• ein Satz von Buchten (500, 501) zum Gruppieren einer Vielzahl der Eiersammler (560), wobei die Buchten (500, 501) an ihren unteren Abschnitten offen sind;
• wobei die Buchten (500, 501) zwischen einem Beladepunkt mit aus den Käfigen entnommenen Sammlern und der Transferlinie für die Neonatenlarven beweglich sind;
• wobei die Transferlinie für die Neonatenlarven im oberen Teil ohne Gehäuse offen ist, um die Buchten (500, 501) im oberen Teil aufzunehmen, und im unteren Teil ein Führungssystem mit ausgeweiteten Wänden (123, 124; 173, 174) aufweist, das als Behälter bezeichnet wird und in seinem oberen Teil eine Breite, die an den Querschnitt der Buchten angepasst ist, und in seinem unteren Teil eine Öffnung (125, 175), die an die Abmessungen des Förderers (110, 160) angepasst ist, aufweist, wobei der Behälter konfiguriert ist, um die Neonatenlarven, die durch Schwerkraft von den Sammlern (560) auf die obere Oberfläche des Förderers (110, 160) fallen, zu gruppieren.

2. Anlage zur Herstellung und Sammlung von Neonatenlarven nach Anspruch 1, **dadurch gekennzeichnet, dass** der Förderer mindestens ein Laufband (110, 160) umfasst, das unter der unteren Öffnung des Behälters mit ausgeweiteten Wänden (123, 124; 173, 174) positioniert ist.

3. Anlage zur Herstellung und Sammlung von Neonatenlarven nach Anspruch 1, **dadurch gekennzeichnet, dass** der Förderer mindestens eine Vibrationsrinne umfasst, die unter der unteren Öffnung des Behälters mit ausgeweiteten Wänden (123, 124; 173, 174) angeordnet ist.

4. Anlage zur Herstellung und Sammlung von Neonatenlarven nach Anspruch 1 oder 2, **dadurch gekennzeichnet, dass** der Behälter mit ausgeweiteten Wänden (123, 124; 173, 174) aus einer geschlitzten Rinne besteht, die zwei seitliche Zuschnitte (123, 124; 173, 174) aufweist, deren untere Ränder durch einen Längsschlitz (125, 175) getrennt sind.

5. Anlage zur Herstellung und Sammlung von Neonatenlarven nach Anspruch 4, **dadurch gekennzeichnet, dass** der Behälter mit ausgeweiteten Wänden (123, 124; 173, 174) im oberen Teil Mittel zur Längsverschiebung der Buchten (500, 501) aufweist.

6. Anlage zur Herstellung und Sammlung von Neonatenlarven nach Anspruch 2, **dadurch gekennzeichnet, dass** die Anlage außerdem ein Ansaugsystem umfasst und dass das Laufband (110, 160) einen horizontalen Hauptabschnitt aufweist, der an seinem stromabwärtigen Ende verlängert ist durch einen geneigten Abschnitt (113, 114) in Richtung des Saugsystems, wobei ein Vibrationsmittel (115) auf den oberen Abschnitt (113) und/oder auf den unteren Abschnitt (113) wirkt.

7. Anlage zur Herstellung und Sammlung von Neonatenlarven nach Anspruch 2, **dadurch gekennzeichnet, dass** das Laufband einen horizontalen Hauptabschnitt aufweist, der an seinem stromabwärtigen Ende durch einen in Richtung des Ansaugsystems geneigten Abschnitt verlängert wird, wobei das untere Band (114) dieses Abschnitts einem Luftstrom ausgesetzt ist, der in die entgegengesetzte Richtung des Laufs des unteren Bandes (114) gerichtet ist.

8. Anlage zur Herstellung und Sammlung von Neonatenlarven nach Anspruch 1, **dadurch gekennzeichnet, dass** sie ein pneumatisches Fördersystem (300) zur Beförderung der Neonatenlarven von der genannten Fördereinrichtung zu einem Mittel zur Verteilung der genannten Neonatenlarven umfasst.

9. Anlage zur Herstellung und Sammlung von Neonatenlarven nach Anspruch 1, **dadurch gekennzeichnet, dass** sie einen Zyklonabscheider (400) umfasst, der von einem vertikalen Zylinder und/oder Kegel gebildet wird, in dem ein aufsteigender Luftstrom erzeugt wird und dessen Boden ein Mittel zur mechanischen und periodischen Entladung umfasst.

10. Anlage zur Herstellung und Sammlung von Neonatenlarven nach dem vorhergehenden Anspruch, **dadurch gekennzeichnet, dass** das genannte mechanische und periodische Entlastungsmittel aus einer Schleuse mit rotierenden Schaufeln besteht.

11. Anlage zur Herstellung und Sammlung von Neonatenlarven nach Anspruch 1, **dadurch gekennzeichnet, dass** sie ein Mittel zur Dosierung der Neonatenmenge durch Wiegen umfasst.

12. Anlage zur Herstellung und Sammlung von Neonatenlarven nach Anspruch 1, **dadurch gekennzeichnet, dass** sie ein Weichenstellungssystem zur Verteilung der gesammelten Neonatenlarven auf die Tabletts eines mehrstufigen Moduls umfasst.

13. Verfahren zur Aufzucht und Sammlung von neonaten Arthropodenlarven, das die folgenden Schritte umfasst:
a) über eine Reihe von mit Insekten gefüllten Käfigen verfügen, die mit mindestens einem Eiersammler versehen sind, in dem die weiblichen Insekten ihre Eier ablegen können;
b) die Neonaten unter dem Einfluss der Schwerkraft zu einem Förderer zu sammeln, der die Neonatenlarven mittels des Förderers zu einem Sammelmittel bewegt; **dadurch gekennzeichnet, dass**
ein Schritt des Gruppierens einer Vielzahl von Sammlern in Buchten (500, 501) und ein Schritt des Bewegens der Buchten (500, 501) auf einer offenen Transferlinie, die eine geschlitzte Rinne mit zwei geneigten Wänden (123, 124; 173, 174) aufweist, die konvergieren und einen Schlitz (125, 175) zum Abgeben der Neonatenlarven in Richtung des Förderers zum stromabwärtigen Ende der Ernte der Neonatenlarven definieren, durchgeführt wird.

14. Verfahren zur Aufzucht und Sammlung von neonaten Arthropodenlarven nach Anspruch 13, **dadurch gekennzeichnet, dass** die Einführung einer neuen Bucht (501) auf der Transferlinie die bereits auf der Transferlinie positionierten Buchten in stromabwärtiger Richtung vorrücken lässt.

## Claims

1. An arthropod rearing facility for the production and collection of neonate larvae, comprising a set of cages provided with at least one egg collector (560) laid by the insects present in said cages, a line for transferring the neonate larvae originating from the hatched eggs from each of said collectors (560) towards a conveyor and equipment for concentrating the neonate larvae originating from said conveyor for conveying towards a rearing module or towards a buffer storage container for neonate larvae, **characterised in that** it further comprises:
• a set of bays (500, 501) for grouping a plurality of said egg collectors (560), said bays (500, 501) being open in their lower portions;
• said bays (500, 501) being movable between a point for loading with collectors removed from the cages, and said neonate larvae transfer line;
• said neonate larvae transfer line being open, without any casing in the upper portion, to receive said bays (500, 501) in the upper portion, and having in the lower portion a guide system with flared walls (123, 124; 173, 174), so-called receptacle, having in its upper portion a width adapted to the section of said bays, and in its lower part an opening (125, 175) adapted to the dimensions of said conveyor (110, 160), said receptacle being configured to group the neonate larvae falling by gravity from said collectors (560) onto the upper surface of said conveyor (110, 160).

2. The facility for the production and collection of neonate larvae according to claim 1, **characterised in that** said conveyor comprises at least one running belt (110, 160), positioned under the lower opening of said receptacle with flared walls (123, 124; 173, 174).

3. The facility for the production and collection of neonate larvae according to claim 1, **characterised in that** said conveyor comprises at least one vibrating corridor positioned under the lower opening of said receptacle with flared walls (123, 124; 173, 174).

4. The facility for the production and collection of neonate larvae according to claim 1 or 2, **characterised in that** said receptacle with flared walls (123, 124; 173, 174) consists of a split gutter having two lateral sidewalls (123, 124; 173, 174) whose lower edges are separated by a longitudinal slot (125, 175).

5. The facility for the production and collection of neonate larvae according to claim 4, **characterised in that** said receptacle with flared walls (123, 124; 173, 174) comprises in the upper portion means for longitudinally moving said bays (500, 501).

6. The facility for the production and collection of neonate larvae according to claim 2, **characterised in that** said facility further comprises a suction system and **in that** said running belt (110, 160) has a horizontal main section extended at its downstream end by an inclined section (113, 114) in the direction of said suction system, a vibrating means (115) acting on the upper section (113) and/or on the lower section (113).

7. The facility for the production and collection of neonate larvae according to claim 2, **characterised in that** said running belt has a horizontal main section extended at its downstream end by a section inclined in the direction of said suction system, the lower belt (114) of this section being subjected to an air flow directed in the direction opposite to the running of said lower belt (114).

8. The facility for the production and collection of neonate larvae according to claim 1, **characterised in that** it comprises a pneumatic transport system (300) for conveying the neonate larvae from said conveyor towards a means for distributing said neonate larvae.

9. The facility for the production and collection of neonate larvae according to claim 1, **characterised in that** it comprises a cyclonic separator (400) formed by a cylinder and/or a vertical cone in which an upward air flow is produced and whose bottom comprises a mechanical and periodic discharge means.

10. The facility for the production and collection of neonate larvae according to the preceding claim, **characterised in that** said mechanical and periodic discharge means consists of a rotary-vane airlock

11. The facility for the production and collection of neonate larvae according to claim 1, **characterised in that** it comprises a means for metering the amount of neonate larvae by weighing.

12. The facility for the production and collection of neonate larvae according to claim 1, **characterised in that** it comprises a switching system for the distribution of neonate larvae collected on the trays of a multi-level module.

13. A method for rearing and collecting neonate larvae of arthropods, comprising the following steps:
a) arranging set of cages filled with insects, provided with at least one egg collector enabling the female insects to lay their eggs;
b) collecting the neonate larvae falling by gravity into a conveyor ensuring the movement of the neonate larvae by means of said conveyor to a collection means; **characterised in that**
it is proceeded with a step of grouping a plurality of collectors in bays (500, 501) and with a step of moving said bays (500, 501) over an open transfer line comprising a split gutter having two convergent inclined walls (123, 124; 173; 174) defining a slot (125, 175) for pouring the neonate larvae towards said conveyor towards the downstream end for harvesting said neonate larvae.

14. The method for rearing and collecting neonate larvae of arthropods according to claim 13, **characterised in that** the introduction of a new bay (501) on said transfer line advances the bays already positioned on said transfer line in a downstream direction.
